# EUROPEAN PATENT APPLICATION

(11) **EP 4 495 203 A1**
(43) Date of publication of application: **22.01.2025**
(21) Application number: 24186300.0
(22) Date of filing: 03.07.2024
(51) Int. Cl.: C09J 183/04, A61B 8/00

(54) **ULTRASOUND COUPLING MATERIAL COMPOSITION, ULTRASOUND COUPLING MATERIAL FILM, AND ULTRASOUND INSPECTION METHOD**

(30) Priority: 12.07.2023 JP 2023114770
(71) Applicant: SHIN-ETSU CHEMICAL CO., LTD., Tokyo 1000005 (JP)
(72) Inventor: Hatakeyama, Jun, Niigata (JP); Ikeda, Joe, Tokyo (JP); Kuroda, Yasuyoshi, Gunma (JP)
(74) Representative: Sonnenhauser, Thomas Martin

(57) **Abstract**

The present invention is an ultrasound coupling material composition, including: a silicone adhesive (A); and a silicone oil (B) having no crosslinking point, wherein the silicone adhesive (A) is a mixture or condensation-reaction product between: a diorganopolysiloxane (a) having a viscosity at 25°C of 100,000 mPa·s or more; and a resin composition (b) including an MQ resin and an organic solvent, and the silicone oil (B) having no crosslinking point has a kinematic viscosity at 25°C within a range of 10 to 100000 mm²/s. This provides: a material that fills roughness on skin to provide high ultrasound transmissibility and sensitivity, has excellent biocompatibility and lightweight, can yield a manufactured ultrasound coupling material film at a low cost, does not considerably decrease the ultrasound transmissibility even with wetted or dried, has softness and excellent flexibility, that causes no residue on the skin after peeling, and causes no rough skin.

## Description

### TECHNICAL FIELD

The present invention relates to an ultrasound coupling material composition, an ultrasound coupling material film, and an ultrasound inspection method.

### BACKGROUND ART

Wearable devices have been developed in recent years with spreading of Internet of Things (IoT). Representative examples of the wearable devices include a watch and glasses that can be connected to the Internet. Wearable devices that can monitor a state of a body at all times are needed in the medical field and the sports field, which are growing fields in future.

For example, proposed is a watch-type wearable device having an optical sensor, an electrostatic capacitance sensor, an ultrasound sensor, a pressure-sensitive sensor, etc. (Patent Document 1).

A wearable device that can perform ultrasound diagnosis has been investigated. In this case, conditions of organs or blood vessels in a body can be visualized to measure a pulse and a blood pressure at all times (Patent Document 2).

With an ultrasound diagnosis device, an ultrasound probe is adhered to skin, ultrasound emitted from a probe surface via an ultrasound transmitter reflects in the body to detect information in the body via the probe with a receiver. During the ultrasound measurement, if the surface to be measured has roughness and the surface of the ultrasound probe cannot cover the roughness on the surface to be measured to allow an air layer to be present, the ultrasound emitted through the ultrasound probe surface reflects on the air layer to fail to reach the body inside. Thus, during the ultrasound diagnosis, a water-soluble gel-based coupling material is inserted between the ultrasound probe surface and the skin (Patent Document 3). Patent Document 4 exemplifies many of the water-soluble coupling materials.

For only once ultrasound measurement, the water-soluble gel-based coupling material is applied on the skin, the ultrasound probe is adhered thereto to perform the measurement, and the gel on the skin surface after the measurement is wiped with water-containing cloth or paper or washed out with water. However, when the ultrasound probe is adhered in a long term to perform the measurement, exercise making the skin sweat, showering, and bathing while the ultrasound probe adhering cause dissolution of the water-soluble gel, resulting in loss of the coupling material to fail to receive the ultrasound signal. Thus, there is a problem that the gel-based coupling material cannot be used for the long-term fitting use.

Accordingly, water-insoluble coupling materials have been proposed. For example, a paraffin-based material (Patent Document 5) and a silicone-gel-based material (Patent Document 6) have been proposed.

The water-soluble gel coupling can be easily removed after the measurement as above. However, removal of paraffin or silicone remaining on the skin needs to wipe with paper or cloth impregnated with paraffin oil etc., which is not convenient. When a silicone oil is added in a silicone gel, which is low-crosslinked silicone rubber, this may cause the silicone oil to remain on the skin, and the remained silicone oil needs to be removed after peeling.

Therefore, a water-insoluble coupling material for the ultrasound measurement has been desired for the long-term fitting use, and there has been a demand for development of a coupling material that causes no residue on the skin after the ultrasound measurement and subsequent peeling of the coupling material from the skin, and that covers roughness on the skin to provide high transmittability and sensitivity of the ultrasound.

### CITATION LIST

### PATENT LITERATURE

Patent Document 1: JP 2021-093127 A
Patent Document 2: WO 2020/049934 A1
Patent Document 3: JP S46-000146 A
Patent Document 4: JP 2005-532871 A
Patent Document 5: JP S63-019135 A
Patent Document 6: US 9211106 B2

### SUMMARY OF INVENTION

### TECHNICAL PROBLEM

The present invention has been made to solve the above problem. An object of the present invention is to provide: a ultrasound coupling material composition that fills roughness on skin to provide high ultrasound transmissibility and sensitivity, that has excellent biocompatibility, that is lightweight, that can yield a manufactured ultrasound coupling material film at a low cost, that does not considerably decrease the ultrasound transmissibility even with wetted or dried, that is soft and excellent in flexibility, that causes no residue on the skin after peeling, and that causes no rough skin; an ultrasound coupling material film; and an ultrasound inspection method.

### SOLUTION TO PROBLEM

To solve the above problem, the present invention provides an ultrasound coupling material composition, comprising:
a silicone adhesive (A); and
a silicone oil (B) having no crosslinking point, wherein
the silicone adhesive (A) is a mixture or condensation reaction product between:
   a diorganopolysiloxane (a) having a viscosity at 25°C of 100,000 mPa·s or more; and
   a resin composition (b) comprising: a resin having a R¹₃SiO_{1/2} unit and a SiO₂ unit, wherein R¹ represents a hydrogen atom or a substituted or unsubstituted monovalent hydrocarbon group having 1 to 10 carbon atoms; and an organic solvent, and
the silicone oil (B) having no crosslinking point has a kinematic viscosity at 25°C within a range of 10 to 100000 mm²/s.

Such an ultrasound coupling material composition is the composition that fills roughness on skin to provide high ultrasound transmissibility and sensitivity, that has excellent biocompatibility, that is lightweight, that can yield a manufactured ultrasound coupling material film at a low cost, that does not considerably decrease the ultrasound transmissibility even with wetted or dried, that is soft and excellent in flexibility, that causes no residue on the skin after peeling, and that causes no rough skin.

In the present invention, the diorganopolysiloxane (a) is preferably a diorganopolysiloxane (a1) having an alkenyl group.

The composition containing such a diorganopolysiloxane (a) allows the alkenyl group to be a crosslinking point, and thereby can form the preferable ultrasound coupling material film.

In this case, the ultrasound coupling material composition preferably further comprises an organohydrogenpolysiloxane having a SiH group as a component (C), and more preferably comprises a platinum-based catalyst.

The diorganosiloxane (a1) having an alkenyl group and the organohydrogenpolysiloxane (C) having a plurality of SiH groups can be crosslinked by an addition reaction with the platinum-based catalyst.

In this case, a value of a number of moles of the SiH group in the component (C) divided by a number of moles of the alkenyl group in the component (A) is preferably within a range of 0.5 to 20.

Such a composition can yield the preferable ultrasound coupling material film by an addition-curing reaction.

In the present invention, the component (B) is preferably a linear or branched diorganopolysiloxane having no alkenyl group and no SiH group.

In the present invention, such a component (B) is preferable.

A value of a mass of the component (B) divided by a total mass of the component (a) and solid contents of the component (b), the component (B), and the component (C) is preferably within a range of 0.2 to 0.8.

Setting the mass of the component (B) to be within such a range can yield the preferable ultrasound coupling material film.

The ultrasound coupling material composition preferably further comprises a dry silica at 0.1 to 10 parts by mass relative to 100 parts by mass of a total amount of the component (a) and solid contents of the component (b), the component (B), and the component (C).

The composition of the present invention containing the dry silica improves strength of the obtained ultrasound coupling material film. Thus, the film hardly tears when removed from the release liner, and easily attached on skin or an ultrasound probe.

The present invention also provides an ultrasound coupling material film comprising a cured product of the above ultrasound coupling material composition.

The ultrasound coupling material film of the present invention is a material that fills roughness on skin to provide high ultrasound transmissibility and sensitivity, that has excellent biocompatibility, that is lightweight, that does not considerably decrease the ultrasound transmissibility even with wetted or dried, that is soft and excellent in flexibility, that causes no residue on the skin after peeling, and that causes no rough skin.

In this case, the ultrasound coupling material film preferably has a film thickness within a range of 10 to 10000 µm.

The film thickness within the above range more enhances the effect of excellent ultrasound transmissibility and sensitivity, lightweight property, excellent flexibility and stretchability, no residue on the skin after peeling, and prevention of rough skin.

The present invention also provides an independent ultrasound coupling material film, wherein the above ultrasound coupling material film is a self-supporting film which is separately isolated from a support.

The ultrasound coupling material film of the present invention can also be used as the self-supporting film (the independent film), and facilitate handling in ultrasound inspection.

The present invention also provides a laminate structure comprising: a support; and the above ultrasound coupling material film directly attached on the support.

The ultrasound coupling material film of the present invention not only can be used as the self-supporting film, but also can be used as the constitution in which the above ultrasound coupling material film is laminated directly on the support (laminate structure), and facilitate handling in ultrasound inspection.

The present invention also provides a method for forming a laminated structure comprising steps of:
applying the above ultrasound coupling material composition on a release liner film as a support, and heat-curing the ultrasound coupling material composition to prepare an ultrasound coupling material film; then
removing the release liner film from the ultrasound coupling material film, and adhering the ultrasound coupling material film onto another support; and
adhering an ultrasound probe with a surface opposite to a surface contacted with the another support to prepare a laminated structure.

In addition, the method may comprise steps of:
applying the above ultrasound coupling material composition on a release liner film as a support, and
heat-curing the ultrasound coupling material composition to prepare an ultrasound coupling material film; and
then removing the release liner film from the ultrasound coupling material film, and adhering the ultrasound coupling material film onto an ultrasound probe to prepare a laminated structure, or alternately applying the above ultrasound coupling material composition on an ultrasound probe, and heat-curing the ultrasound coupling material composition to form an ultrasound coupling material film and prepare a laminated structure.

In the present invention, the ultrasound coupling material composition can be applied on the release liner film or an ultrasound probe, and heat-cured for use. Thus, in ultrasound inspection, a desired aspect can be selected as above to prepare the laminated structure.

The present invention also provides an inspection method comprising the above method for forming a laminated structure, and subsequently contacting the ultrasound probe on skin to perform ultrasound inspection so that a surface with the formed ultrasound coupling material film is directed to the skin, wherein the method excludes a method for operating, treating, or diagnosing a human.

The ultrasound coupling material film formed by using the ultrasound coupling material composition of the present invention has excellent water resistance while having excellent ultrasound transmittability and sensitivity, and causes no residue on skin after measurement and can reduce subject's load. Thus, the ultrasound coupling material film is extremely useful in various ultrasound inspection such as long-term fitting use.

### ADVANTAGEOUS EFFECTS OF INVENTION

As above, the ultrasound coupling material composition is a material that fills roughness on skin to provide high ultrasound transmissibility and sensitivity, that has excellent biocompatibility, that is lightweight, that can be manufactured at a low cost, that does not considerably decrease the ultrasound transmissibility even with wetted or dried, that is soft and excellent in flexibility, that causes no residue on the skin after peeling, and that causes no rough skin.

### BRIEF DESCRIPTION OF DRAWINGS

FIG. 1 is a schematic sectional view illustrating an example of a coupling material film of the present invention;
FIG. 2 is a schematic sectional view illustrating another example of a coupling material film of the present invention;
FIG. 3 is a schematic sectional view illustrating an example of use of adhering a coupling material film of the present invention onto skin;
FIG. 4 is a schematic sectional view illustrating an example of use of adhering a coupling material film of the present invention onto an ultrasound probe;
FIG. 5 is a schematic sectional view illustrating an example of use of adhering a coupling material film of the present invention between an ultrasound probe and skin;
FIG. 6 is a schematic sectional view illustrating an example of performing ultrasound diagnosis in a state of adhering a coupling material film of the present invention between an ultrasound probe and skin;
FIG. 7 is a schematic sectional view illustrating an example of performing ultrasound diagnosis in a state of adhering a conventional coupling material film between an ultrasound probe and skin;
FIG. 8 is a photograph of adhering a coupling material film of the present invention onto an arm;
FIG. 9 is a photograph in which a grease of a water-soluble gel of Comparative Example 5 is dispensed on an arm;
FIG. 10 is a photograph of a handy-type ultrasound diagnosis meter (device) viewed from an ultrasound probe surface;
FIG. 11 is a photograph in which the ultrasound coupling material film in a sectional view state of FIG. 1 adheres to an ultrasound probe while peeling off a release liner;
FIG. 12 is a photograph in which an ultrasound measurement device is mounted on a coupling material film to perform ultrasound diagnosis;
FIG. 13 is a photograph of an ultrasonogram when an ultrasound signal is finely received; and
FIG. 14 is a photograph of an ultrasonogram when an ultrasound signal is defectively received.

### DESCRIPTION OF EMBODIMENTS

As noted above, there has been a demand for development of: a coupling material composition that is water-insoluble, that has excellent filling ability for roughness on skin, excellent sound transmissibility and biocompatibility, that is lightweight, that can be manufactured at a low cost, that is soft and has flexibility and strength, and that can stably collect an ultrasound signal in contact with skin for a long term even with dried or wetted by bathing or sweating; a coupling laminate film thereof; and an ultrasound measurement method thereof.

Coupling materials being water-insoluble and having excellent sound transmittability and biocompatibility include silicone materials. The aforementioned Patent Document 6 describes a coupling material using a silicone gel. The silicone gel is an extremely soft silicone gel with ultralow crosslinking density. However, only softness is insufficient for filling micrometer-order fine roughness of soft skin.

Methods for further softening the silicone gel include a method of adding a non-crosslinkable silicone oil or a hydrocarbon-based mineral oil, as described in JP 2015-028178 A. The addition of the non-crosslinkable silicone oil improves filling ability for roughness on skin to improve sensitivity of the ultrasound measurement when used as the ultrasound coupling material. However, addition of a large amount of the non-crosslinkable silicone oil causes bleed-out to remain the residual silicone oil on skin when the ultrasound measurement is finished and the coupling material film is peeled from the skin. A residue of the silicone oil requires wiping the skin with towel or absorbent cotton impregnated with mineral oil to remove the residue, which is not preferable due to labor.

Accordingly, the present inventors have earnestly studied the above problem, and consequently invented that a silicone adhesive is used as a base instead of the silicone gel in order to prevent bleed-out even when a large amount of the non-crosslinkable silicone oil is added. This silicone adhesive is a reaction product or mixture of a crosslinkable silicone and an MQ resin having a R¹₃SiO_{1/2} unit and a SiO₂ unit, and prevents the non-crosslinkable silicone oil from bleed-out. The present inventors have found that the above problem can be solved by such an ultrasound coupling material composition containing the above silicone adhesive and the silicone oil having no crosslinking point. This finding has led to completion of the present invention.

Specifically, the present invention is an ultrasound coupling material composition comprising:
a silicone adhesive (A); and
a silicone oil (B) having no crosslinking point, wherein
the silicone adhesive (A) is a mixture or condensation reaction product between:
   a diorganopolysiloxane (a) having a viscosity at 25°C of 100,000 mPa·s or more; and
   a resin composition (b) comprising: a resin having a R¹₃SiO_{1/2} unit and a SiO₂ unit, wherein R¹ represents a hydrogen atom or a substituted or unsubstituted monovalent hydrocarbon group having 1 to 10 carbon atoms; and an organic solvent, and
the silicone oil (B) having no crosslinking point has a kinematic viscosity at 25°C within a range of 10 to 100000 mm²/s.

As above, the present invention relates to: the coupling material film inserted between skin and an ultrasound diagnosis meter which is used for contacting with the skin of a living body, visualizing conditions of organs and blood vessels in the body, and measuring a pulse and a blood pressure; the coupling material composition providing the film; and a method for manufacturing the coupling material film.

Hereinafter, the present invention will be described in detail, but the present invention is not limited thereto.

### <Ultrasound Coupling Material Composition>

The ultrasound coupling material composition of the present invention comprises: a silicone adhesive; a silicone oil having no crosslinking point; and an organic solvent.

In the ultrasound coupling material composition of the present invention, the silicone adhesive (A) is preferably a mixture of: a mixture or a condensation reaction product between a component (a) and a component (b); and an organic solvent. The component (a) is a diorganopolysiloxane, and the component (b) is composed of a resin having a R¹₃SiO_{1/2} unit and a SiO₂ unit and an organic solvent. The ultrasound coupling material composition preferably contains: the silicone oil component (B) having no crosslinking point and having a kinematic viscosity at 25°C within a range of 10 to 100000 mm²/s; an organohydrogenpolysiloxane component (C); and a platinum-based catalyst. R¹ represents a hydrogen atom or a substituted or unsubstituted monovalent hydrocarbon group having 1 to 10 carbon atoms.

Hereinafter, each component will be described in more detail.

### Component (A)

The silicone adhesive (A) is a mixture or condensation reaction product between: a diorganopolysiloxane (a) having a viscosity at 25°C of 100,000 mPa·s or more; and a resin composition (b) comprising: a resin having a R¹₃SiO_{1/2} unit and a SiO₂ unit, wherein R¹ represents a hydrogen atom or a substituted or unsubstituted monovalent hydrocarbon group having 1 to 10 carbon atoms; and an organic solvent.

### Component (a)

The component (a) blended in the ultrasound coupling material composition of the present invention is a diorganopolysiloxane having a viscosity at 25°C of 100,000 mPa·s or more. The diorganopolysiloxane is not particularly limited as long as it has such a property, but preferably a diorganopolysiloxane (a1) having an alkenyl group. As the diorganopolysiloxane having an alkenyl group, diorganopolysiloxanes corresponding to a component (A1) described in JP 2015-193803 A and having a viscosity at 25°C of 100,000 mPa·s or more may be used.

Examples of the alkenyl group include a vinyl group, an allyl group, a hexenyl group, an octenyl group, an acryloylpropyl group, an acryloylmethyl group, a methacryloylpropyl group, and a vinyloxypropyl group, and a vinyl group is particularly preferably used.

The group other than the alkenyl group is a substituted or unsubstituted monovalent hydrocarbon group having 1 to 10 carbon atoms. Specific examples thereof include: alkyl groups, such as a methyl group, an ethyl group, a propyl group, and a butyl group; cycloalkyl groups, such as a cyclohexyl group; and aryl groups, such as a phenyl group or a tolyl group; and groups in which a part or all of hydrogen atoms bonded to a carbon atom in these groups are substituted with other groups such as halogen, an amino group, a hydroxy group, or a cyano group, for example, a 3-aminopropyl group, a 3,3,3-trifluoropropyl group, a 3-hydroxypropyl group, and a 3-cyanopropyl group. The group is particularly preferably a methyl group and a phenyl group.

The component (a) may further contain a diorganopolysiloxane (a2) corresponding to a component (A2) described in JP 2015-193803 A, having a viscosity at 25°C of 100,000 mPa·s or more, and having no alkenyl group. The terminals of the polymers A1 and A2 are a hydroxy group or an alkoxy group, and may be bonded to the component (b) by a condensation reaction between the group and a silanol group in the MQ resin of the component (b). In this case, the component (a) and the component (b) are not independent components but form an integrated component. The condensation reaction may be performed by using an acid catalyst or an alkaline catalyst with heating.

The polysiloxanes a1 and a2 may be linear or branched.

Examples of the adhesive silicone-based resin include addition-reaction curable resins or radical-crosslinking-reaction curable resins. With the addition-curable type, the alkenyl group and the SiH group are crosslinked by an addition reaction, and meanwhile with the radical-curable type, the crosslinking proceeds not only in the presence of the alkenyl group but also in the absence of the alkenyl group. In this case, crosslinking with the silicone oil component (B) having no crosslinking group may occur. This crosslinking may deteriorate flowability of the silicone oil to deteriorate filling ability on skin, and may deteriorate sensitivity as the ultrasound coupling material. Therefore, the addition-curable type is preferred to the radical-crosslinking type.

As the component (a), a modified siloxane having a group selected from an amino group, an oxirane group, an oxetane group, a polyether group, a hydroxy group, a carboxy group, a mercapto group, a methacryl group, an acryl group, a phenol group, a silanol group, a carboxylic anhydride group, an aryl group, an aralkyl group, an amide group, an ester group, and a lactone ring may be added. The modified siloxane may be a siloxane in which any one of one terminal, both terminals, and a side chain thereof is modified.

It is effective to reduce amounts of octamethylcyclotetrasiloxane and decamethylcyclopentasiloxane in the component (a) for reducing skin irritation. A method for reducing the amounts is described in JP 2015-193803 A.

The components (a1) and (a2) may be any kind of oil or raw rubber. When a viscosity of the raw rubber is as high as more than 500,000 mPa-s, a viscosity of a solution in which the raw rubber is dissolved with toluene at a concentration of 30 mass% (30-mass% dissolution viscosity) is preferably 100,000 mPa·s or less. The 30 mass% dissolution viscosity is further preferably 1,000 to 60,000 mPa·s. A 30-mass% dissolution viscosity of more than 100,000 mPa·s causes the composition to have an excessively high viscosity, resulting in difficulty in stirring during manufacture. In the present invention, the viscosity (absolute viscosity) is a value at 25°C measured by a rotary viscosimeter.

### Component (b)

The component (b) is a resin composition comprising: a resin having a R¹₃SiO_{1/2} unit and a SiO₂ unit, wherein R¹ represents a hydrogen atom or a substituted or unsubstituted monovalent hydrocarbon group having 1 to 10 carbon atoms; and an organic solvent.

The resin in the component (b) is a so-called MQ resin having a R¹₃SiO_{1/2} unit (M unit) and a SiO₂ unit (Q unit), and has a form of dissolution in the organic solvent. R¹ represents a hydrogen atom or a substituted or unsubstituted monovalent hydrocarbon group having 1 to 10 carbon atoms. For the organic solvent to dissolve the MQ resin, an organic solvent described later is appropriately selected.

R¹ represents a hydrogen atom or a substituted or unsubstituted monovalent hydrocarbon group having 1 to 10 carbon atoms, and examples thereof include: alkyl groups, such as a methyl group, an ethyl group, a propyl group, and a butyl group; cycloalkyl groups; aryl groups, such as a phenyl group; and alkenyl groups, such as a vinyl group, an allyl group, and a hexenyl group. Examples thereof further include groups in which a part or all of hydrogen atoms bonded to a carbon atom in these groups are substituted with other groups such as halogen, an amino group, a hydroxy group, or a cyano group, for example, a 3-aminopropyl group, a 3,3,3-trifluoropropyl group, a 3-hydroxypropyl group, and a 3-cyanopropyl group.

A number ratio, R¹₃SiO_{1/2} unit / SiO₂ unit, is preferably 0.6 to 1.7. The number ratio of the lower limit or more does not deteriorate adhesive force and tackiness, and the number ratio of the upper limit or less yields good adhesive force and retainability.

The component (b) may have an OH group, and an OH group content is preferably 0.01 to 4.0 mass% relative to a total mass of the component (B). The OH group content of the lower limit or more yields high aggregation force of the adhesive, and the OH group content of the upper limit or less does not deteriorate tackiness of the adhesive. The MQ resin of the component (b) typically has a silanol group, and this silanol group can cause a condensation reaction with the hydroxy group terminal or the alkoxy group terminal of the diorganopolysiloxane in the component (a) to form a chemical bond.

The component (b) may be used in combination of two or more. The component (b) may contain a R¹SiO_{3/2} unit (T unit) and/or a R¹₂SiO unit (D unit) as necessary.

The presence of the component (b) exhibits adhesiveness, and prevents the bleed-out of the component (B) and remaining of the component (B) on skin after peeling the coupling material.

A solid-content ratio in the component (a) and the component (b) (mass ratio in the component (A) excluding the organic solvent) can be 20 to 100 parts by mass of the component (a) and 1 to 20 parts by mass of the component (b) relative to 120 parts by total mass of the component (A).

### Component (B)

The component (B) is a silicone oil having no crosslinking point (non-crosslinkable type), and having a kinematic viscosity at 25°C within a range of 10 to 100000 mm²/s, more preferably 50 to 50000 mm²/s. In the present invention, the kinematic viscosity is a measured value at 25°C with an Ostwald viscosimeter.

The component (B) as a single form is a flowable oil, and adding the component (B) can fill fine roughness on skin to improve ultrasound transmittability when the ultrasound coupling material film adheres to the skin.

The non-crosslinkable silicone oil of the component (B) is not particularly limited as long as it has the kinematic viscosity at 25°C within the above range, but preferably a linear or branched diorganopolysiloxane having no alkenyl group and no SiH group. Such a component (B) has good compatibility with the silicone adhesive (A), and particularly, causes no bleed-out even when a large amount of the non-crosslinkable silicone oil is added. Therefore, the bleed out of the oil can be prevented.

A value of the mass of the component (B) divided by a total mass of the component (a), the solid content of the component (b), the component (B), and the component (C) (total mass of the solid contents of the components (A) to (C)) is preferably within a range of 0.2 to 0.8 (0.20 to 0.80), and further preferably within a range of 0.3 to 0.78.

### Component (C)

The component (C) is an organohydrogenpolysiloxane having a SiH group as the component (C). When the silicone adhesive has the alkenyl group, the alkenyl group and the SiH group are crosslinked (addition-cured) due to an addition reaction (hydrosilylation reaction), and in this case, the component (C) can function as a crosslinking agent. The component (C) is not particularly limited as long as it is the organohydrogenpolysiloxane having a SiH group, and a component (C) described in JP 2015-193803 A, which has two or more, preferably three or more SiH groups in the molecule, may be used, for example.

A viscosity at 25°C of the component (C) is preferably 1 to 1,000 mPa.s, and further preferably 2 to 500 mPa·s. The component (C) may be a mixture of two or more thereof. The component (C) may have a linear, branched, or cyclic structure. A number of the SiH groups is preferably two or more in one molecule of the organohydrogenpolysiloxane. The number is more preferably three or more. When the composition is the addition-curable type, such a component (C) is preferable. When the MQ resin of the component (b) has the SiH group, the component (C) is not necessarily needed.

The diorganosiloxane (a1) having the alkenyl group and the organohydrogenpolysiloxane (C) having a plurality of the SiH groups can be crosslinked by an addition reaction with a platinum-based catalyst. A ratio of a number of moles of the SiH groups divided by a number of moles of the alkenyl groups is preferably within a range of 0.5 to 20, and more preferably within a range of 1 to 15.

Examples of the platinum-based catalyst include: platinum-based catalysts such as chloroplatinic acid, an alcohol solution of chloroplatinic acid, a reaction product of chloroplatinic acid and an alcohol, a reaction product of chloroplatinic acid and an olefin compound, a reaction product of chloroplatinic acid and a vinyl-group-containing siloxane, a platinum-olefin complex, and a platinum-vinyl-group-containing siloxane complex; and platinum-group metal-based catalysts such as a rhodium complex and a ruthenium complex. A catalyst in which these catalysts are dissolved or dispersed in an alcohol, hydrocarbon, or siloxane solvent may also be used.

An addition amount of the platinum-based catalyst is preferably within a range of 5 to 2,000 ppm, and particularly preferably within a range of 10 to 500 ppm in terms of metal mass, relative to 100 parts by mass of the resin solution combining (A), (B), (C), and the organic solvent (the ultrasound coupling material composition).

When the addition-curable type silicone resin is used, an addition-reaction controlling agent may be added. This addition-reaction controlling agent is added as a quencher in order to prevent the platinum-based catalyst from action in the solution and under a low-temperature environment after forming the applied film and before the heating curing. Specific examples thereof include 3-methyl-1-butyn-3-ol, 3-methyl-1-pentyn-3-ol, 3,5-dimethyl-1-hexyn-3-ol, 1-ethynylcyclohexanol, 3-methyl-3-trimethylsiloxy-1-butyne, 3-methyl-3-trimethylsiloxy-1-pentyne, 3,5-dimethyl-3-trimethylsiloxy-1-hexyne, 1-ethynyl-1-trimethylsiloxycyclohexane, bis(2,2-dimethyl-3-butynoxy)dimethylsilane, 1,3,5,7-tetramethyl-1,3,5,7-tetravinylcyclotetrasiloxane, and 1,1,3,3-tetramethyl-1,3-divinyldisiloxane.

An addition amount of the addition-reaction controlling agent is preferably within a range of 0 to 10 parts by mass, and particularly preferably 0.05 to 3 parts by mass relative to 100 parts by mass of the resin solution.

### Silica Particles

Into the composition of the present invention, dry silica particles may be added. Adding the dry silica improves strength of the ultrasound coupling material film. Thus, the film hardly tears when removed from the release liner, and can be easily attached on skin or an ultrasound probe. The dry silica has a specific surface area within a range of 50 to 500 m²/g, and preferably 100 to 400 m²/g. The specific surface area is measured by a BET method. A silica in which silanol on the silica surface is substituted with a trimethylsilyl group, a dimethylsilyl group, or dimethylsilicone is preferable because of high dispersibility. For the dry silica, a commercially available product may be used, and for example, AEROSIL RX200 (manufactured by NIPPON AEROSIL CO., LTD.) may be used.

As for an addition amount of the dry silica, the dry silica is preferably added at 0.1 to 10 parts by mass relative to 100 parts by mass of the total of the component (a), the solid content of the component (b), the component (B), and the component (C) (total of the solid contents of the components (A) to (C)).

### Organic Solvent

Into the composition of the present invention, an organic solvent same as or different from the organic solvent contained in the component (b) may be further added. Regulating a type and amount of the organic solvent contained in the composition of the present invention can yield good operability.

The organic solvent is not particularly limited, and examples thereof include the followings. Specific examples thereof include: aromatic hydrocarbon solvents, such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; and aliphatic hydrocarbon solvents, such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-triethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0^{2,7}]undec-4-ene, n-dodecane, n-tridecane, n-pentadecane, n-hexadecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffin. These may be any mixture.

The hydrocarbon solvent is preferably an aliphatic solvent not containing an aromatic solvent, and more specifically, preferably mainly any one of normal paraffins, isoparaffins, and naphthenes, or a mixture thereof. The normal paraffins and isoparaffins have little odor compared with naphthenes, and thus use of the normal paraffins, the isoparaffins, or a mixture of them is preferable because load to environment and human body can be reduced while reducing the odor. Here, the normal paraffins, the isoparaffins, or a mixture of them means a highly purified solvent containing the normal paraffins, the isoparaffins, or a mixture of them as a main component, but even these extremely highly purified solvent is contaminated with a trace amount of naphthenes and aromatic substances at a concentration from a ppm level to a maximum 0.5% or less (based on mass). However, an effect of the trace amount of these components to the odor is significantly small, which is negligible. Therefore, the normal paraffins, the isoparaffins, or a mixture of them herein means a solvent substantially not containing the naphthenes and aromatic substances at an amount equal to or larger than the above trace amount impurity level.

Commercially available organic solvents may also be used, and the solvent may be: a branched paraffin solvent mixture such as "ISOPAR^{™} M" and "ISOPAR^{™} G" (Exxon Mobil Corporation) and "IP Solvent 2028" (Idemitsu Kosan Co., Ltd.), specifically an isoparaffin hydrocarbon; or a naphthene hydrocarbon such as "Exxsol^{™} D110" (Exxon Mobil Corporation) and "AF Solvent No. 4" (ENEOS). The solvents of ISOPAR(R) series, which are the isoparaffin solvents, are preferable. These solvents have features of low reactivity, being stabile, low toxicity, high safety, and little odor.

An addition amount of the organic solvent is preferably within a range of 10 to 50,000 parts by mass relative to 100 parts by mass of the total of the component (a) and the resin (b) (the solid content of the resin composition (b)).

### Other Additives

In the ultrasound coupling material composition of the present invention, a wet silica, alumina particles, titania particles, zirconia particles, and a pigment may be mixed in addition to the dry silica. Addition of the wet silica particles, the alumina particles, the titania particles, or the zirconia particles improves strength. When the composition is colored, the pigment is added. The alumina particles, the titania particles, and the zirconia particles may be preferably used in any of dry and wet types, and particles having surfaces treated with hydrophobic silylation agent have excellent dispersibility. The shape of the wet silica, the alumina particles, the titania particles, and the zirconia particles may be any of spherical, oval, irregular-shaped, hollow, and porous shapes.

### <Ultrasound Coupling Material Film>

The ultrasound coupling material film of the present invention comprises a cured product of the above ultrasound coupling material composition.

In the present invention, an ultrasound coupling material film is formed on a support such as a release liner film by using the above ultrasound coupling material composition, and this film is inserted between skin and an ultrasound probe to transmit the ultrasound between the skin and the ultrasound probe.

Hereinafter, the ultrasound coupling material (film) of the present invention will be described in detail with reference to the drawings, but the present invention is not limited thereto.

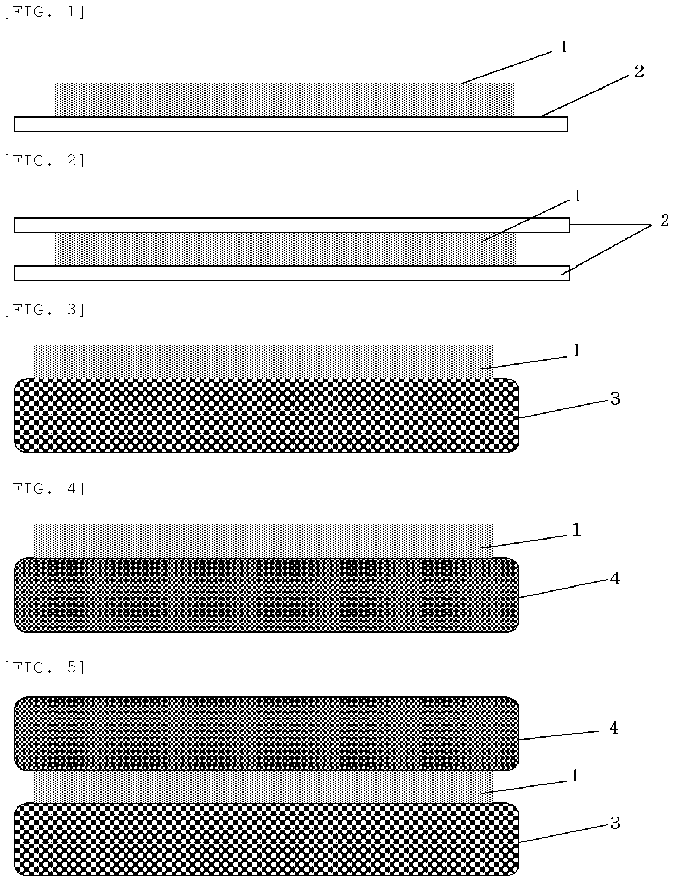

FIG. 1 is a schematic sectional view illustrating an example of the ultrasound coupling material film of the present invention. An ultrasound coupling material 1 in FIG. 1 illustrates an embodiment of being formed on a release liner (support) 2.

As a substrate of the release liner, paper, plastic film made of plastic, glass, metal, cloth, etc. is selected. Examples of the paper include woodfree paper, coated paper, art paper, glassine paper, polyethylene-laminated paper, craft paper, Japanese paper, and synthetic paper. Examples of the plastic film include polyethylene film, polypropylene film, polyester film, polyimide film, polyamide film, polyvinyl chloride film, polyvinylidene chloride film, polyvinyl alcohol film, polycarbonate film, polytetrafluoroethylene film, polystyrene film, ethylene-vinyl acetate copolymer film, ethylene-vinyl alcohol copolymer film, triacetylcellulose film, polyether ether ketone film, and polyphenylene sulfide film. As for the glass, a thickness, a type, etc. thereof are not particularly limited, and the glass may be subjected to a chemically reinforcing treatment. Glass fiber may also be applied, and the glass fiber may be used singly, or may form a composite with another resin for use. Examples of the cloth include natural fabric cloth, synthetic fabric cloth, and artificial leather. Examples of the metal include aluminum foil, copper foil, gold foil, silver foil, and nickel foil. Among these, the paper and the plastic film are preferable, and the polyester film is particularly preferable.

On the substrate of the release liner, a fluorine-based releasing agent described in JP 2020-100763 A or JP 2020-100764 A may be applied, which can reduce releasing force.

### <Method for Manufacturing Ultrasound Coupling Material Film>

The method for manufacturing the ultrasound coupling material film of the present invention is not particularly limited, and manufacturing examples will be described with reference to the drawings hereinafter.

First, the ultrasound coupling material composition of the present invention is applied on a support (substrate) such as the release liner. A method for applying the ultrasound coupling material composition on the substrate such as the release liner is not particularly limited, but methods such as dip coating, spray coating, spin coating, bar coating, comma coating, slit coating, roll coating, flow coating, doctor coating, screen printing, flexography, gravure printing, and inkjet printing are preferable, for example.

Then, the ultrasound coupling material composition applied on the support (substrate) is cured.

A method for curing the ultrasound coupling material composition is not particularly limited, and the film after the applying is preferably cured with either of heat or light, or a combination thereof, for example.

A temperature for the heating is not particularly limited, and preferably approximately 50 to 250°C, for example. Examples of the heating methods include hot wind, electric heating such as a hotplate, and heat generation such as irradiation with infrared ray or microwave.

When the heating and the light irradiation, the infrared irradiation, or the microwave irradiation are combined, the heating and the light irradiation, the infrared irradiation, or the microwave irradiation may be simultaneously performed, the heating may be performed after the light irradiation, the infrared irradiation, or the microwave irradiation, or the light irradiation, the infrared irradiation, or the microwave irradiation may be performed after the heating. Before the heating after the applying, air drying may be performed for the purpose of evaporating the solvent.

A film thickness of the heated and cured ultrasound coupling material film is preferably 10 to 10000 um, more preferably within a range of 10 to 4000 um, and further preferably within a range of 20 to 3000 um. Within each of the numerical range of the film thickness, an upper limit thereof may be appropriately 5000 um, 2000 um, 500 um, or 400 um, a lower limit thereof may be appropriately 30 um, 50 um, 100 um, or 120 um, and these may be appropriately combined.

The ultrasound coupling material composition may not be applied on the release liner but applied on the ultrasound probe.

As illustrated in FIG. 2, a structure in which the ultrasound coupling material film 1 is vertically sandwiched between the release liners 2 can be formed. This configuration facilitates transportation of the adhesive ultrasound coupling material film.

### <Usage of Ultrasound Coupling Material Film>

As illustrated in FIG. 3, the release liner 2 on one side shown in FIG. 2 is peeled, and the ultrasound coupling material film 1 can adhere onto skin 3.

As illustrated in FIG. 4, the ultrasound coupling material film 1 can adhere onto an ultrasound probe 4 instead.

A structure during ultrasound diagnosis is the ultrasound coupling material film 1 sandwiched between the skin 3 and the ultrasound probe 4, as illustrated in FIG. 5.

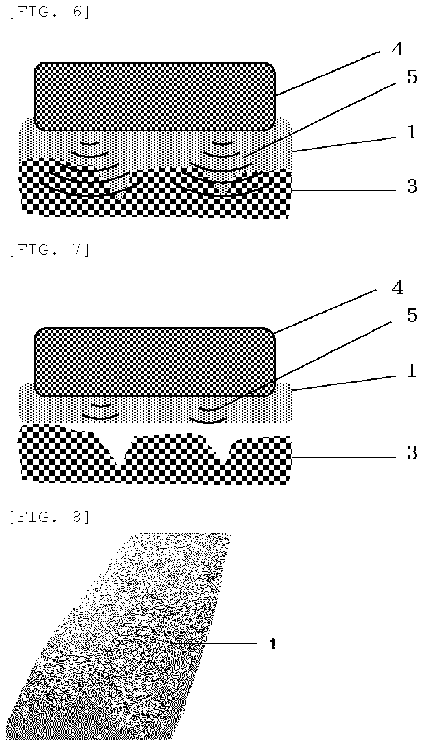

It is described by using FIGS. 6 and 7 that ultrasound emitted from the ultrasound probe is transmitted into the skin via the ultrasound coupling material film during ultrasound diagnosis. A frequency of the ultrasound is typically sound with a frequency of 3 MHz or higher, used for ultrasound diagnosis.

FIG. 6 is a schematic sectional view of a state where the ultrasound coupling material film 1 is sandwiched between the skin 3 and the ultrasound probe 4. The ultrasound coupling material film 1 fills roughness on the skin 3, and thereby ultrasound 5 emitted from the ultrasound probe is transmitted through the skin.

Meanwhile, FIG. 7 is also a schematic sectional view of a state where the ultrasound coupling material film 1 is sandwiched between the skin 3 and the ultrasound probe 4. However, the ultrasound coupling material film 1 does not fill roughness on the skin 3 in this case, and thereby ultrasound 5 is not transmitted through the skin.

FIG. 8 is a photograph of a state where one side of the release liner is peeled, the ultrasound coupling material film 1 is adhered onto skin of an inside of a forearm, and the other release liner is peeled.

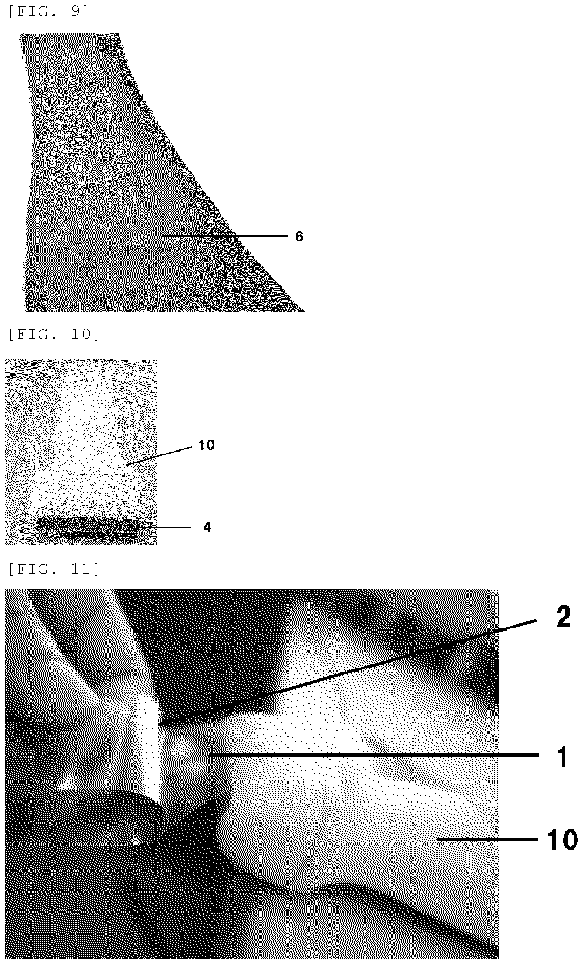

FIG. 9 is a photograph of a state where a water-soluble ultrasound coupling gel (water-soluble gel coupling material 6) is dispensed from a tube on skin of an inside of a forearm. After the dispensing, the gel is spread with a finger to form the ultrasound coupling material film.

FIG. 10 is a photograph of a handy-type ultrasound diagnosis meter 10 viewed from the ultrasound probe 4 side.

FIG. 11 is a photograph in which the ultrasound coupling material film 1 adheres onto the ultrasound probe of the ultrasound diagnosis meter 10 while peeling the release liner 2. It is found that the ultrasound coupling material film is an independent film.

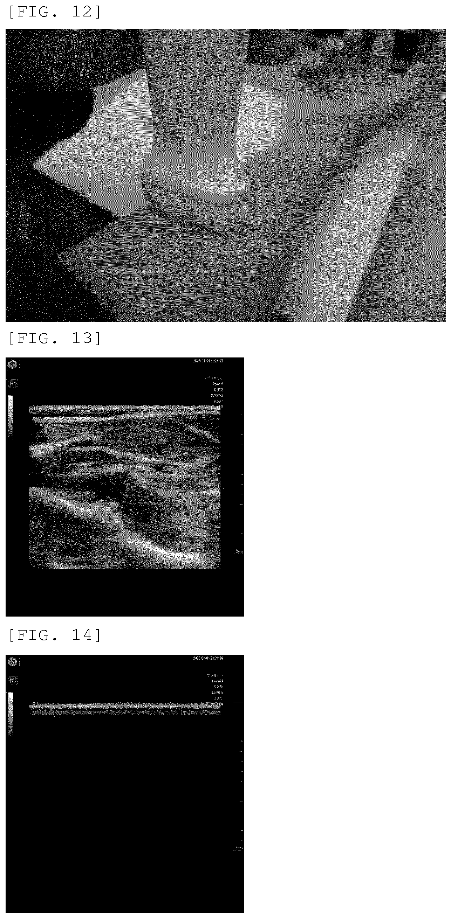

FIG. 12 is a photograph in which a handy-type ultrasound diagnosis meter is mounted on the coupling material film to measure an ultrasonogram.

FIGS. 13 and 14 are measured ultrasonograms.

The ultrasound diagnosis is performed by adhering the ultrasound probe onto skin to allow ultrasound emitted from the probe surface via an ultrasound emitter to be reflected, and detecting information in a body with a receiver via the probe. The ultrasound transmitted through the body inside and reflected on a target portion can yield the ultrasonogram as shown in FIG. 13. Meanwhile, if an air layer is present during the ultrasound measurement due to roughness on a surface to be measured, etc., the ultrasound emitted from the ultrasound probe surface is reflected on the air layer to fail to reach the body inside, resulting in an unclear ultrasonogram as shown in FIG. 14.

That is, when the ultrasound coupling material film 1 fills roughness on the skin 3 in a state where the ultrasound coupling material film 1 is sandwiched between the skin 3 and the ultrasound probe 4 as in FIG. 6, the ultrasound 5 is transmitted in the skin and consequently yields the ultrasonogram as shown in FIG. 13.

Meanwhile, the ultrasound coupling material film 1 does not fill the roughness on the skin 3 as in FIG. 7, the ultrasound 5 is not transmitted in the skin and consequently yields the ultrasonogram as shown in FIG. 14.

### EXAMPLE

Hereinafter, the present invention will be specifically described by using Examples and Comparative Examples, but the present invention is not limited thereto. When a measurement target has a viscosity higher than 500,000 mPa·s, hereinafter, described is a viscosity (30% dissolution viscosity) in which the measurement target is dissolved in toluene in the concentration of 30 mass%. That is, the viscosity described as the 30% dissolution viscosity means that the viscosity of the measurement target is more than 500,000 mPa·s. The above absolute viscosity is a value at 25°C measured with a rotary viscosimeter.

### Examples 1 to 12 and Comparative Examples 1 to 5

Each component used in Examples and Comparative Examples will be described below.

### (Component A (Silicone Adhesive))

### (Component a)

A vinyl-group-containing polydimethylsiloxane having a viscosity as a 30-mass% toluene solution of 27,000 mPa-s, having an alkenyl-group content of 0.02 mol/100-g, and having molecular chain terminals blocked with a SiMe₂Vi group was used as a siloxane compound 1.

A vinyl-group-containing polydimethylsiloxane having a viscosity as a 30-mass% toluene solution of 27,000 mPa-s, having an alkenyl-group content of 0.01 mol/100-g, and having molecular chain terminals blocked with a SiMe₂Vi group was used as a siloxane compound 2.

Both of viscosities at 25°C of the siloxane compounds 1 and 2 as it is are 100,000 mPa·s or more.

### (Component b)

A 60-mass% toluene solution of a polysiloxane of an MQ resin composed of a Me₃SiO_{0.5} unit and a SiO₂ unit (Me₃SiO_{0.5} unit / SiO₂ unit = 0.8) was used as an MQ resin 1.

A 60-mass% hexane solution of a polysiloxane of an MQ resin composed of a Me₃SiO_{0.5} unit and a SiO₂ unit (Me₃SiO_{0.5} unit / SiO₂ unit = 0.8) was used as an MQ resin 2.

A 60-mass% isoparaffin-solvent ISOPAR(R) G solution of a polysiloxane of an MQ resin composed of a Me₃SiO_{0.5} unit and a SiO₂ unit (Me₃SiO_{0.5} unit / SiO₂ unit = 0.8) was used as an MQ resin 3.

### (Component A (Silicone Adhesive))

A solution was composed of: 40 parts by mass of a vinyl-group-containing polydimethylsiloxane having 42,000 mPa·s of viscosity as a 30-mass% toluene solution, having an alkenyl-group content of 0.02 mol/100-g, and having molecular chain terminals blocked with OH group; 100 parts by mass of a 60 mass% toluene solution of a polysiloxane being an MQ resin composed of a Me₃SiO_{0.5} unit and a SiO₂ unit (Me₃SiO_{0.5} unit / SiO₂ unit = 0.8) ; and 26.7 parts by mass of toluene. This solution was heated for 4 hours with reflux, and then cooled to obtain a product with the polydimethylsiloxane bonded to the MQ resin. This product was used as a siloxane compound 3.

### (Component B)

As a non-crosslinkable silicone oil, KF-96, manufactured by Shin-Etsu Chemical Co., Ltd., shown in Table 1 was used. A kinematic viscosity thereof at 25°C corresponds to a number directly before "CS" in the Table. For example, "KF-96 1,000CS" has a kinematic viscosity of 1,000 mm²/s, and "KF-96 100CS" has a kinematic viscosity of 100 mm²/s. The kinematic viscosity is a measurement value at 25°C with an Ostwald viscosimeter.

### (Component C)

As methylhydrogensilicone oil, KF-99 and KF-9901, manufactured by Shin-Etsu Chemical Co., Ltd., were used.

### (Additive)

Dry silica: AEROSIL RX200 (manufactured by NIPPON AEROSIL CO., LTD., subjected to a surface treatment with a trimethylsilyl group)

### (Organic Solvent)

ISOPAR^{™} G (available from Exxon Mobil Corporation)

A coupling material solution (ultrasound coupling material composition) described in Table 1 was mixed. Into 100 parts by mass of this solution, 1 part by mass of a platinum catalyst PL-56, manufactured by Shin-Etsu Chemical Co., Ltd., was added, and the mixture was further mixed.

### [Table 1]

| Ultrasound coupling material solution | Component a (parts by mass) | Component b (parts by mass) | Component A (integrated a-b) (parts by mass) | Component B (parts by mass) | Component C (parts by mass) | B/(a+b solid contents + B + c) (%) | SiH/Vi | Additive (parts by mass) | Organic solvent (parts by mass) |
|---|---|---|---|---|---|---|---|---|---|
| Ultrasound coupling material solution 1 | Siloxane compound 1 (100) | MQ resin 1 (20) | - | KF-96 1,000es (80) | KF-99 (10) | 39.6 | 8.3 | - | Toluene (66.7) |
| Ultrasound coupling material solution 2 | Siloxane compound 1 (100) | MQ resin 1 (20) | - | KF-96 1,000es (50) | KF-99 (10) | 29.1 | 8.3 | - | Toluene (66.7) |
| Ultrasound coupling material solution 3 | Siloxane compound 1 (100) | MQ resin 1 (20) | - | KF-96 1,000CS (120) | KF-99 (10) | 49.6 | 8.3 | AEROSIL RX200 (1.5) | Toluene (66.7) |
| Ultrasound coupling material solution 4 | Siloxane compound 1 (100) | MQ resin 1 (20) | - | KF-96 1,000es (180) | KF-99 (10) | 59.6 | 8.3 | AEROSIL RX200 (1.5) | Toluene (66.7) |
| Ultrasound coupling material solution 5 | Siloxane compound 1 (100) | MQ resin 1 (20) | - | KF-96 1,000CS (280) | KF-99 (10) | 69.7 | 8.3 | AEROSIL RX200 (1.5) | Toluene (66.7) |
| Ultrasound coupling material solution 6 | Siloxane compound 1 (100) | MQ resin 1 (20) | - | KF-96 100CS (50) | KF-99 (10) | 29.1 | 8.3 | - | Toluene (66.7) |
| Ultrasound coupling material solution 7 | Siloxane compound 1 (100) | MQ resin 1 (20) | - | KF-96 10,000es (300) | KF-99 (10) | 71.1 | 8.3 | AEROSIL RX200 (1.5) | Toluene (66.7) |
| Ultrasound coupling material solution 8 | Siloxane compound 2 (100) | MQ resin 1 (20) | - | KF-96 1,000CS (280) | KF-99 (10) | 69.7 | 8.3 | AEROSIL RX200 (1.5) | Toluene (66.7) |
| Ultrasound coupling material solution 9 | - | | Siloxane compound 3 (120) | KF-96 1,000CS (180) | KF-99 (10) | 68.7 | 8.3 | AEROSIL RX200 (1.5) | - |
| Ultrasound coupling material solution 10 | Siloxane compound 1 (100) | MQ resin 2 (20) | - | KF-96 1,000CS (280) | KF-99 (10) | 69.7 | 8.3 | AEROSIL RX200 (1.5) | Hexane (66.7) |
| Ultrasound coupling material solution 11 | Siloxane compound 1 (100) | MQ resin 3 (20) | - | KF-96 1,000CS (280) | KF-99 (10) | 69.7 | 8.3 | AEROSIL RX200 (1.5) | ISOPAR^{™} G (66.7) |
| Ultrasound coupling material solution 12 | Siloxane compound 2 (100) | MQ resin 3 (20) | - | KF-96 1,000es (280) | KF-9901 (12) | 69.7 | 8.6 | AEROSIL RX200 (1.5) | ISOPAR^{™} G (66.7) |
| Comparative ultrasound coupling material solution 1 | Siloxane compound 1 (100) | MQ resin 1 (20) | - | - | KF-99 (10) | 0 | 8.3 | - | Toluene (66.7) |
| Comparative ultrasound coupling material solution 2 | Siloxane compound 1 (100) | - | - | KF-96 1,000es (80) | KF-99 (10) | 92.1 | 8.3 | - | Toluene (66.7) |
| Comparative ultrasound coupling material solution 3 | Siloxane compound 1 (100) | MQ resin 1 (20) | - | KF-96 100,000es (80) | KF-99 (10) | 39.6 | 8.3 | - | Toluene (66.7) |
| Comparative ultrasound coupling material solution 4 | Siloxane compound 1 (100) | MQ resin 1 (20) | - | KF-96 5CS (80) | KF-99 (10) | 39.6 | 8.3 | - | Toluene (66.7) |

### (Formation of Coupling Material Film)

The above ultrasound coupling material solution was applied on a release liner of a PET separator SS1A (thickness: 50 um), manufactured by NIPPA CORPORATION, with a bar coater, dried with air for 10 minutes, baked with a hot-air oven at 125°C for 10 minutes, and then cured to form the coupling material film illustrated in FIG. 1. The cured film was covered with a release liner of a PET separator SS1A (thickness: 50 um), manufactured by NIPPA CORPORATION, to produce the ultrasound coupling material film sandwiched with the two release liners as illustrated in FIG. 2.

### (Comparative Example 5)

In Comparative Example 5, a conventional grease-like water-soluble gel coupling material 6 was used as the ultrasound coupling material.

### (Measurement of Thickness of Bio-Contacting Layer)

A thickness of the ultrasound coupling material film prepared above was measured by using a micrometer. Table 2 shows the results.

One of the two release liners was peeled, the surface of the ultrasound coupling material film was adhered onto skin of a forearm, and the other release liner was peeled after the adhesion onto the skin to form the ultrasound coupling material film on the skin as illustrated in FIG. 8.

As for the gel ultrasound coupling material of Comparative Example 5, the grease-like water-soluble gel coupling material 6 was adhered onto an arm as illustrated in FIG. 9, and spread with a finger to form a smooth film. The film thickness of the coupling material film failed to be measured.

### (Measurement of Ultrasound Signal)

As a handy-type ultrasound diagnosis device (ultrasound diagnosis meter) used for measuring an ultrasound signal, SONON 500L, manufactured by Healcerion Co., Ltd., was used. As illustrated in FIG. 12, a probe surface of the ultrasound diagnosis device was contacted with the ultrasound coupling material film on the arm, and ultrasound diagnosis was performed with a frequency of 8.5 MHz.

The ultrasound diagnosis was performed with adhesion of the coupling material on the arm, then the arm was immersed in water for one minute, then the arm was dried, and the ultrasound diagnosis was performed again. Thereafter, the coupling material film was peeled from the skin, and whether a residue was present or not on the skin was visually checked.

### (Evaluation of Water Resistance)

In the ultrasound diagnosis after the immersion in water, a case where a clear image as shown in the above

FIG. 13 was exhibited was judged as good, and a case of an unclear image as of FIG. 14 was judged as bad.

Table 2 shows the evaluation results of the ultrasound coupling materials described in Table 1.

**[Table 2]**

| Example | Ultrasound coupling material solution | Thickness of ultrasound coupling material (µm) | Ultrasonogram | Ultrasonogram after water immersion | Residue on skin after removing coupling material |
|---|---|---|---|---|---|
| Example 1 | Ultrasound coupling material solution 1 | 133 | Good | Good | Absent |
| Example 2 | Ultrasound coupling material solution 2 | 140 | Good | Good | Absent |
| Example 3 | Ultrasound coupling material solution 3 | 130 | Good | Good | Absent |
| Example 4 | Ultrasound coupling material solution 4 | 136 | Good | Good | Absent |
| Example 5 | Ultrasound coupling material solution 5 | 121 | Good | Good | Absent |
| Example 6 | Ultrasound coupling material solution 6 | 120 | Good | Good | Absent |
| Example 7 | Ultrasound coupling material solution 7 | 123 | Good | Good | Absent |
| Example 8 | Ultrasound coupling material solution 8 | 139 | Good | Good | Absent |
| Example 9 | Ultrasound coupling material solution 9 | 133 | Good | Good | Absent |
| Example 10 | Ultrasound coupling material solution 10 | 141 | Good | Good | Absent |
| Example 11 | Ultrasound coupling material solution 11 | 140 | Good | Good | Absent |
| Example 12 | Ultrasound coupling material solution 12 | 130 | Good | Good | Absent |
| Comparative Example 1 | Comparative bio-electrode solution 1 | 110 | Bad | Bad | Absent |
| Comparative Example 2 | Comparative bio-electrode solution 2 | 106 | Good | Good | Present |
| Comparative Example 3 | Comparative bio-electrode solution 3 | 129 | Bad | Bad | Absent |
| Comparative Example 4 | Comparative bio-electrode solution 4 | 101 | Good | Good | Present |
| Comparative Example 5 | Water-soluble gel coupling material | - | Good | Bad | Absent |

Table 2 shows the results in case of adhering the ultrasound coupling material films shown in Examples 1 to 12 on the skin, in which the silicone oil of the component (B) was added into the silicone adhesive composed of the component (a) and the component (b). As shown in Table 2, this case yielded the good ultrasonograms, yielded the similar images even when the arm was immersed in water, and caused no residue on the skin after peeing the ultrasound coupling material film from the skin. Meanwhile, the silicone adhesive of Comparative Example 1 which added no component (B) yielded the bad image with the ultrasound diagnosis. The film shown in Comparative Example 2, which contained the silicone gel without MQ resin of the component (b), yielded the good ultrasonogram but caused residue of the added silicone oil of the component (B) after peeling from the skin. Comparative Example 3, which contained the component (B) having an excessively large kinematic viscosity, caused the bad ultrasonogram. In contrast, Comparative Example 4, which contained the component (B) having an excessively small kinematic viscosity, yielded the good ultrasonogram but caused residue after peeling the ultrasound coupling material film from the skin. The conventional water-soluble gel coupling material of Comparative Example 5 yielded the good ultrasonogram after applying the coupling material, but caused dissolution of the coupling material when the arm was immersed in water, resulting in the bad ultrasonogram.

The ultrasound coupling material of the present invention can fill roughness on skin to yield a good ultrasonogram (that is, high ultrasound transmissibility and sensitivity), and is excellent in biocompatibility and lightweight because the silicone is main component. The ultrasound coupling material composition (solution) of the present invention can be applied on a support as necessary, cured etc. to manufacture an ultrasound coupling material film at a low cost. This ultrasound coupling material film does not contain a water-soluble material, and thereby does not considerably decrease the ultrasound transmissibility even with wetted or dried. The ultrasound coupling material film is a cured product derived from the silicone adhesive (A), the film is soft and excellent in flexibility, and causes no residue on the skin after peeling and causes no rough skin. As above, the ultrasound coupling material composition of the present invention has the excellent features.

The present description includes the following embodiments.
[1]: An ultrasound coupling material composition, comprising:
   a silicone adhesive (A); and
   a silicone oil (B) having no crosslinking point, wherein
   the silicone adhesive (A) is a mixture or condensation reaction product between:
      a diorganopolysiloxane (a) having a viscosity at 25°C of 100,000 mPa·s or more; and
      a resin composition (b) comprising: a resin having a R¹₃SiO_{1/2} unit and a SiO₂ unit, wherein R¹ represents a hydrogen atom or a substituted or unsubstituted monovalent hydrocarbon group having 1 to 10 carbon atoms; and an organic solvent, and
   the silicone oil (B) having no crosslinking point has a kinematic viscosity at 25°C within a range of 10 to 100000 mm²/s.
[2]: The ultrasound coupling material composition of the above [1], wherein the diorganopolysiloxane (a) is a diorganopolysiloxane (a1) having an alkenyl group.
[3]: The ultrasound coupling material composition of the above [2], further comprising an organohydrogenpolysiloxane having a SiH group as a component (C).
[4]: The ultrasound coupling material composition of the above [3], further comprising a platinum-based catalyst.
[5]: The ultrasound coupling material composition of the above [4], wherein a value of a number of moles of the SiH group in the component (C) divided by a number of moles of the alkenyl group in the component (A) is within a range of 0.5 to 20.
[6]: The ultrasound coupling material composition of any one of the above [2] to [5], wherein the component (B) is a linear or branched diorganopolysiloxane having no alkenyl group and no SiH group.
[7]: The ultrasound coupling material composition of any one of the above [3] to [6], wherein a value of a mass of the component (B) divided by a total mass of the component (a) and solid contents of the component (b), the component (B), and the component (C) is within a range of 0.2 to 0.8.
[8]: The ultrasound coupling material composition of any one of the above [3] to [7], further comprising a dry silica at 0.1 to 10 parts by mass relative to 100 parts by mass of a total amount of the component (a) and solid contents of the component (b), the component (B), and the component (C).
[9] An ultrasound coupling material film, comprising a cured product of the ultrasound coupling material composition of any one of the above [1] to [8].
[10]: The ultrasound coupling material film of the above [9], wherein the ultrasound coupling material film has a film thickness within a range of 10 to 10000 um.
[11]: An independent ultrasound coupling material film, wherein the ultrasound coupling material film of the above [9] or [10] is a self-supporting film which is separately isolated from a support.
[12]: A laminate structure, comprising:
   a support; and
   the ultrasound coupling material film of the above [9] or [10] directly attached on the support.
[13]: A method for forming a laminated structure, comprising steps of:
   applying the ultrasound coupling material composition of any one of the above [1] to [8] on a release liner film as a support, and heat-curing the ultrasound coupling material composition to prepare an ultrasound coupling material film; then
   removing the release liner film from the ultrasound coupling material film, and adhering the ultrasound coupling material film onto another support; and
   adhering an ultrasound probe with a surface opposite to a surface contacted with the another support to prepare a laminated structure.
[14]: A method for forming a laminated structure, comprising steps of:
   applying the ultrasound coupling material composition of any one of the above [1] to [8] on a release liner film as a support, and heat-curing the ultrasound coupling material composition to prepare an ultrasound coupling material film; and then
   removing the release liner film from the ultrasound coupling material film, and adhering the ultrasound coupling material film onto an ultrasound probe to prepare a laminated structure.
[15]: A method for forming a laminated structure, comprising a step of:
   applying the ultrasound coupling material composition of any one of the above [1] to [8] on an ultrasound probe, and heat-curing the ultrasound coupling material composition to form an ultrasound coupling material film and prepare a laminated structure.
[16]: An inspection method, comprising the method for forming a laminated structure of any one of the above [13] to [15], and subsequently contacting the ultrasound probe on skin to perform ultrasound inspection so that a surface with the formed ultrasound coupling material film is directed to the skin, wherein the method excludes a method for operating, treating, or diagnosing a human.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. An ultrasound coupling material composition, comprising:
a silicone adhesive (A); and
a silicone oil (B) having no crosslinking point, wherein
the silicone adhesive (A) is a mixture or condensation reaction product between:
a diorganopolysiloxane (a) having a viscosity at 25°C of 100,000 mPa·s or more; and
a resin composition (b) comprising: a resin having a R¹₃SiO_{1/2} unit and a SiO₂ unit, wherein R¹ represents a hydrogen atom or a substituted or unsubstituted monovalent hydrocarbon group having 1 to 10 carbon atoms; and an organic solvent, and
the silicone oil (B) having no crosslinking point has a kinematic viscosity at 25°C within a range of 10 to 100000 mm²/s.

2. The ultrasound coupling material composition according to claim 1, wherein the diorganopolysiloxane (a) is a diorganopolysiloxane (a1) having an alkenyl group.

3. The ultrasound coupling material composition according to claim 2, further comprising an organohydrogenpolysiloxane having a SiH group as a component (C).

4. The ultrasound coupling material composition according to claim 3, further comprising a platinum-based catalyst.

5. The ultrasound coupling material composition according to claim 4, wherein a value of a number of moles of the SiH group in the component (C) divided by a number of moles of the alkenyl group in the component (A) is within a range of 0.5 to 20.

6. The ultrasound coupling material composition according to any one of claims 2 to 5, wherein the component (B) is a linear or branched diorganopolysiloxane having no alkenyl group and no SiH group.

7. The ultrasound coupling material composition according to any one of claims 3 to 6, wherein a value of a mass of the component (B) divided by a total mass of the component (a) and solid contents of the component (b), the component (B), and the component (C) is within a range of 0.2 to 0.8.

8. The ultrasound coupling material composition according to any one of claims 3 to 7, further comprising a dry silica at 0.1 to 10 parts by mass relative to 100 parts by mass of a total amount of the component (a) and solid contents of the component (b), the component (B), and the component (C).

9. An ultrasound coupling material film, comprising a cured product of the ultrasound coupling material composition according to any one of claims 1 to 8.

10. The ultrasound coupling material film according to claim 9, wherein the ultrasound coupling material film has a film thickness within a range of 10 to 10000 µm.

11. An independent ultrasound coupling material film, wherein the ultrasound coupling material film according to claim 9 or 10 is a self-supporting film which is separately isolated from a support.

12. A laminate structure, comprising:
a support; and
the ultrasound coupling material film according to claim 9 or 10 directly attached on the support.

13. A method for forming a laminated structure, comprising steps of:
A)
applying the ultrasound coupling material composition according to any one of claims 1 to 8 on a release liner film as a support, and heat-curing the ultrasound coupling material composition to prepare an ultrasound coupling material film; then
removing the release liner film from the ultrasound coupling material film, and adhering the ultrasound coupling material film onto another support; and
adhering an ultrasound probe with a surface opposite to a surface contacted with the another support to prepare a laminated structure; or
B)
applying the ultrasound coupling material composition according to any one of claims 1 to 8 on a release liner film as a support, and heat-curing the ultrasound coupling material composition to prepare an ultrasound coupling material film; and then
removing the release liner film from the ultrasound coupling material film, and adhering the ultrasound coupling material film onto an ultrasound probe to prepare a laminated structure; or
C)
applying the ultrasound coupling material composition according to any one of claims 1 to 8 on an ultrasound probe, and heat-curing the ultrasound coupling material composition to form an ultrasound coupling material film and prepare a laminated structure.

14. An inspection method, comprising the method for forming a laminated structure according to any one of claims 13A to 13C, and subsequently contacting the ultrasound probe on skin to perform ultrasound inspection so that a surface with the formed ultrasound coupling material film is directed to the skin, wherein the method excludes a method for operating, treating, or diagnosing a human.
